# EUROPÄISCHE PATENTSCHRIFT

(11) **EP 1 153 913 B1**
(45) Veröffentlichungstag und Bekanntmachung des Hinweises auf die Patenterteilung: **13.08.2003**
(21) Anmeldenummer: 01110731.5
(22) Anmeldetag: 03.05.2001
(51) Int. Cl.: C07C 213/08, C07C 213/06

(54) **Verfahren zur Herstellung von Esterquats**
Process for the preparation of esterquats
Procédé de préparation d'esters quaternaires

(30) Priorität: 11.05.2000 DE 10022966
(43) Veröffentlichungstag der Anmeldung: 14.11.2001
(73) Patentinhaber: Cognis Iberia, S.L., 08755 Castellbisbal, (Barcelona) (ES)
(72) Erfinder: Bigorra Llosas, Joaquin, Dr., 08203 Sabadell (ES); Bonastre Nuria, Gilabert, Dr., 08210 Barbera del Vall (ES); Pi Subirana, Rafael, Dr., 08400 Granollers (ES)
(74) Vertreter: Fabry, Bernd, Dr.

(56) Entgegenhaltungen:
- EP-A- 0 284 036
- EP-A- 0 708 075
- EP-A- 1 006 103
- DE-C- 4 409 322

## Beschreibung

### Gebiet der Erfindung

Die Erfindung befindet sich auf dem Gebiet der Kationtenside und betrifft ein verbessertes Verfahren zur Herstellung von Esterquats ausgehend von Triglyceriden.

### Stand der Technik

Unter der Bezeichnung "Esterquats" werden im allgemeinen quatemierte Fettsäurealkanolaminestersalze verstanden, die sich in den letzten Jahren neben den bekannten Tetraalkylammoniumsalzen als weitere Gruppe kationischer Tenside für den Einsatz als Textilweichmacher und Konditioniermittel für die Kosmetik durchgesetzt haben. Üblicherweise erfolgt ihre Herstellung durch Kondensation von Fettsäuren mit Alkanolaminen und nachfolgende Quaternierung der Ester mit Methylchlorid oder Dimethylsulfat. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quatemiert. Aus der Deutschen Patentschrift **DE 4308794 C1** (Henkel) ist überdies ein Verfahren zur Herstellung fester Esterquats bekannt, bei dem man die Quaternierung von Triethanolaminestern in Gegenwart von geeigneten Dispergatoren, vorzugsweise Fettalkoholen, durchführt. Übersichten zu diesem Thema sind beispielsweise von R.Puchta et al. in **Tens.Surf.Det., 30, 186 (1993),** M.Brock in **Tens.Surf.Det, 30, 394 (1993), R.Lagerman et al. in J.Am. Oil.Chem.Soc., 71, 97 (1994) sowie I.Shapiro in Cosm.Toil. 109, 77 (1994)** erschienen.

Ein besonders effektives Verfahren zur Herstellung von Esterquats wird in der Europäischen Patentschrift **EP 0750606 B1** (Cognis) vorgeschlagen. Anstelle der Fettsäuren werden hier Triglyceride eingesetzt, die mit den Alkanolaminen zu den Fettsäurealkanolaminestern umgeestert und dann quaterniert werden. Der Vorteil des Verfahrens besteht vor allem darin, dass man von niedrig veredelteren Rohstoffen ausgeht. Nachteilig ist jedoch, dass die auf diesem Wege erhaltenen Esterquats im Vergleich zu Produkten mit einer vergleichbaren Fettsäurezusammensetzung, die durch Veresterung gewonnen wurden, schlechtere anwendungstechnische Eigenschaften aufweisen. Dies hat dazu geführt, dass sich die Umesterungsprodukte trotz der niedrigeren Herstellkosten bislang nicht haben durchsetzen können.

Die Aufgabe der vorliegenden Erfindung hat somit darin bestanden, das Verfahren der **EP 0750606 B1** so zu verbessern, dass in ihren anwendungstechnischen Eigenschaften konkurrenzfähige Produkte erhalten werden, ohne dass der Vorteil in den Herstellkosten verlorengeht.

### Beschreibung der Erfindung

Gegenstand der Erfindung ist ein Verfahren zur Herstellung von Esterquats, bei dem man Triglyceride mit Alkanolaminen umestert und die resultierenden Fettsäurealkanolaminester anschließend in Gegenwart von Lösungsmitteln mit Alkylhalogeniden oder Dialkylsulfaten quaterniert, welches sich dadurch auszeichnet, dass man während der Umesterung freiwerdendes Glycerin kontinuierlich aus dem Reaktionsgleichgewicht entfernt.

In umfangreichen Untersuchungen hat die Anmelderin gefunden, dass die Nachteile in den anwendungstechnischen Eigenschaften der Umesterungsprodukte auf Anteile von Partialglyceriden zurückzuführen sind. Durch die simple Maßnahme, das bei der Umesterung freiwerdende Glycerin kontinuierlich aus dem Reaktionsgemisch zu entfernen und so die Quelle für Partialglyceride zu eliminieren, werden nun auch auf Basis von Triglyceriden Esterquats erhalten, die in ihren Eigenschaften den Veresterungsprodukten nicht länger nachstehen. Die zusätzlichen Aufwendungen für den Destillationsschritt führen zu keiner nennenswerten Belastung der Herstellkosten, zumal das abgetrennte Glycerin ganz oder anteilig als Lösungsmittel für die Ester in der Quatemierung dienen kann.

### Umesterung

Zur Herstellung der Esterquats kann grundsätzlich von beliebigen synthetischen Triglyceriden oder natürlichen Fetten und Ölen ausgegangen werden, welche der allgemeinen Formel **(I)** folgen, 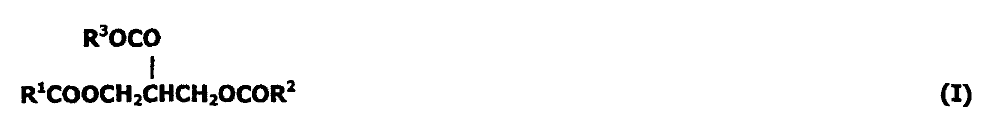 in der R¹CO, R²CO und R³CO unabhängig von einander für lineare und/oder verzweigte, gesättigte und/oder ungesättigte Acylreste mit 6 bis 22, vorzugsweise 12 bis 18 Kohlenstoffatomen und 0 und/oder 1 bis 3 Doppelbindungen stehen. Vorzugsweise sind die Triglyceride gehärtet oder wenigstens teilgehärtet, d.h. weisen Iodzahlen von 0 bzw. 0,5 bis 40 und insbesondere 5 bis 25 auf. Als Einsatzstoffe eignen sich beispielsweise Palmkernöl, Olivenöl und dergleichen, wobei der Einsatz von Rindertalg, Kokosöl und Palmöl, gegebenenfalls nach einem Härtungsschritt, bevorzugt ist. Als Alkanolamine kommen vor allem Triethanolamin (TEA) oder Methyldiethanolamin (MDA) sowie deren Gemische in Frage. Die Umesterung findet in an sich bekannter Weise statt, wobei vorwiegend alkalische Katalysatoren wie etwa Alkalihydroxide, Alkalialkoholate, Zinkseifen und dergleichen verwendet werden. Zur Herstellung der quatemierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestem mit einem durchschnittlichen Veresterungsgrad von 1,5 bis 1,9 dar.

### Quaternierung

Auch die Quaternierung kann in an sich bekannter Weise durchgeführt werden, wobei es sich bewährt hat, die Alkanolaminester zuvor in ein geeignetes Lösungsmittel einzubringen. Vorzugsweise kommen hierfür niedere aliphatische Alkohole, wie beispielsweise Isopropylalkohol oder Ethylenglycol in Frage, die aus anwendungstechnischer Sicht nicht stören und auch bei kosmetischer Anwendung der Produkte unbedenklich sind. In einer bevorzugten Ausführungsform der Erfindung wird das bei der Umesterung freigesetzte und abgetrennte Glycerin wieder in die Quaternierung als Lösungsmittel für die Ester zurückgeführt. Auf diese Weise können die zusätzlichen Kosten für den Destillationsschritt weitgehend aufgefangen werden. Als Quaternierungsmittel kommen Alkalihalogenide, wie z.B. Methylchlorid oder Dialkylsulfate, wie z.B. Dimethylsulfat in Frage. Für weitere Einzelheiten zur Durchführung von Umesterung und Quaternierung sei auf die schon eingangs erwähnte EP 0750606 B1 verwiesen, auf deren gesamten Inhalt ausdrücklich Bezug genommen wird.

### Beispiele

**Beispiel 1.** In einer 2-1-Destillationsapparatur wurden 780 g (0,92 Mol) Rindertalg, 175 g (1,17 Mol) Triethanolamin und 1,4 g Kaliumhydroxid in Form einer 45 Gew.-%igen wäßrigen Lösung vorgelegt und auf 80 °C erwärmt. Anschließend wurde Vakuum angelegt und der Druck bis auf 2 mmHg abgesenkt, während die Temperatur kontinuierlich bis auf 180 °C gesteigert wurde. Anschließend wurden weitere 75 g (0,5 Mol) Triethanolamin zugegeben. Im Verlauf von 2 h wurden 81,5 g (= 96 % der Theorie) Glycerin abdestilliert. Danach wurde das Vakuum gebrochen, und die Mischung abgekühlt und 200 g (0,34 Mol) des entstandenen Talgfettsäuretriethanolaminesters in eine Rührapparatur überführt. Dort wurde der Ester in einer Mischung aus 18 g Glycerin und 46 g Isopropylalkohol gelöst und unter Rühren bei 65 °C über einen Zeitraum von 4 h portionsweise mit 41 g (0,32 Mol) Dimethylsulfat versetzt. Die Esterquatzubereitung wies einen Trockenrückstand von 85 Gew.-% auf.

**Beispiel 2.** Analog Beispiel 1 wurde ein Talgfettsäuretriethanolaminester hergestellt und in 63 g Isopropylalkohol gelöst. Die resultierende Esterquatzubereitung wies einen Trockenrückstand von 90 Gew.-% auf.

## Patentansprüche

1. Verfahren zur Herstellung von Esterquats, bei dem man Triglyceride mit Alkanolaminen und die resultierenden Fettsäurealkanolaminester anschließend in Gegenwart von Lösungsmitteln mit Alkylhalogeniden oder Dialkylsulfaten quaterniert, **dadurch gekennzeichnet, dass** man während der Umesterung freiwerdendes Glycerin kontinuierlich aus dem Reaktionsgleichgewicht entfernt.

2. Verfahren nach Anspruch 1, **dadurch gekennzeichnet, dass** man Triglyceride der Formel **(I)** einsetzt, 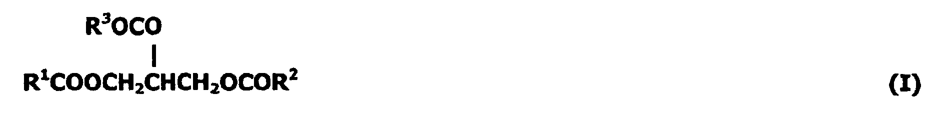 in der R¹CO, R²CO und R³CO unabhängig von einander für lineare und/oder verzweigte, gesättigte und/oder ungesättigte Acylreste mit 6 bis 22 Kohlenstoffatomen und 0 und/oder 1 bis 3 Doppelbindungen stehen.

3. Verfahren nach den Ansprüchen 1 und/oder 2, **dadurch gekennzeichnet, dass** man als Triglyceride Rindertalg, Kokosöl oder Palmöl einsetzt.

4. Verfahren nach mindestens einem der Ansprüche 1 bis 3, **dadurch gekennzeichnet, dass** man als Alkanolamine Triethanolamin (TEA) und/oder Methyldiethanolamin (MDA) einsetzt.

5. Verfahren nach mindestens einem der Ansprüche 1 bis 3, **dadurch gekennzeichnet, dass** man die Triglyceride und die Alkanolamine - bezogen auf die Fettsäuregruppen - im molaren Verhältnis 1,1 : 1 bis 3 : 1 einsetzt.

6. Verfahren nach mindestens einem der Ansprüche 1 bis 4, **dadurch gekennzeichnet, dass** man das bei der Umesterung abgetrennte Glycerin ganz oder teilweise als Lösungsmittel in der Quatemierung einsetzt.

## Claims

1. A process for the production of esterquats in which triglycerides are transesterified with alkanolamines and the resulting fatty acid alkanolamine esters are subsequently quaternized with alkyl halides or dialkyl sulfates in the presence of solvents, **characterized in that** glycerol released during the transesterification is continuously removed from the reaction equilibrium.

2. A process as claimed in claim 1, **characterized in that** triglycerides corresponding to formula **(I)**: 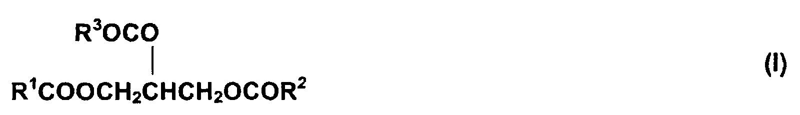 in which R¹CO, R²CO and R³CO independently of one another represent linear and/or branched, saturated and/or unsaturated acyl groups containing 6 to 22 and preferably 12 to 18 carbon atoms and 0 and/or 1 to 3 double bonds,
are used.

3. A process as claimed in claims 1 and/or 2, **characterized in that** bovine tallow, coconut oil or palm oil is used as the triglyceride.

4. A process as claimed in at least one of claims 1 to 3, **characterized in that** triethanolamine (TEA) and/or methyl diethanolamine (MDA) is/are used as the alkanolamines.

5. A process as claimed in at least one of claims 1 to 3, **characterized in that** the triglycerides and the alkanolamines are used in a molar ratio of 1.1:1 to 3:1, based on the fatty acid groups.

6. A process as claimed in at least one of claims 1 to 4, **characterized in that** the glycerol removed during the transesterification is completely or partly used as solvent in the quaternization step.

## Revendications

1. Procédé de préparation d'esterquats dans lequel on transestérifie des triglycérides avec des alkanol amines et on quaternise ensuite les esters d'alkanolamine d'acide gras résultants en présence de solvants, avec des halogénures d'alkyle ou des sulfates d'alkyle,
**caractérisé en ce qu'**
on élimine pendant la transestérification le glycérol libéré en continu de l'équilibre réactionnel.

2. Procédé selon la revendication 1,
**caractérisé en ce qu'**
on met en oeuvre des triglycérides de formule (I) 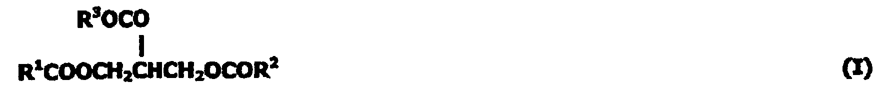 dans laquelle R¹CO, R²CO et R³CO indépendamment l'un de l'autre, représentent des restes acyle linéaires et/ou ramifiés, saturés et/ou non saturés ayant de 6 à 22 atomes de carbone et 0 et/ou de 1 à 3 double liaisons.

3. Procédé selon les revendications 1 et/ou 2,
**caractérisé en ce qu'**
on met en oeuvre comme triglycérides, du suif de bovins, de l'huile de coco ou de l'huile de palme.

4. Procédé selon au moins l'une des revendications 1 à 3,
**caractérisé en ce qu'**
on met en oeuvre comme alkanoïdes de la triéthanol amine (TEA) et/ou de la méthyl-diéthanolamine (MDA).

5. Procédé selon au moins une des revendications 1 à 3,
**caractérisé en ce qu'**
on met en oeuvre les triglycérides et les alkanolamines - rapporté aux groupes acide gras - dans un rapport molaire de 1,1 : 1 à 3 : 1.

6. Procédé selon au moins une des revendications 1 à 4,
**caractérisé en ce qu'**
on met en oeuvre le glycérol séparé dans la transestérification totalement ou partiellement, comme solvant dans la quaternisation.
